# EUROPEAN PATENT APPLICATION

(11) **EP 1 990 411 A1**
(43) Date of publication of application: **12.11.2008**
(21) Application number: 07831746.8
(22) Date of filing: 13.11.2007
(51) Int. Cl.: C12N 15/09, C12Q 1/68

(54) **METHOD OF ORGANISM IDENTIFICATION BY DNA TAG**

(30) Priority: 22.01.2007 JP 2007011739
(71) Applicant: Japan Software Management Co., Ltd., Kanagawa-ku Yokohama-shi Kanagawa 221-0056 (JP); Igarashi, Takao, Totsuka-ku Yokohama-shi Kanagawa 244-0813 (JP); Nasu, Hisanori, Izumi-ku Yokohama-shi Kanagawa 245-0018 (JP)
(72) Inventor: NASU, Hisanori, Yokohama-shi Kanagawa 245-0018 (JP); TSUJIMOTO, Atsumi, Yokohama-shi Kanagawa 221-0056 (JP); IMAMURA, Kaoru, Yokohama-shi Kanagawa 221-0056 (JP); KOBAYASHI, Takanori, Yokohama-shi Kanagawa 231-0802 (JP); GOJOUBORI, Takashi, Mishima-shi Shizuoka 411-0043 (JP)
(74) Representative: Woods, Geoffrey Corlett
(86) International application number: PCT/JP2007/072016
(87) International publication number: WO 2008/090664

(57) **Abstract**

The object is to provide a novel organism identification method which can be used as an alternative method to a conventional organism identification method which utilizes DNA and requires enormous labor. Developed is an organism identification method for determining whether or not an organism of interest is identical to a specific organism or a progeny thereof, which is **characterized by** introducing a DNA tag(s) in advance into a cell(s) of the specific organism, or a parasite or a symbiont with the specific organism, and determining whether or not the organism of interest is identical to the specific organism or a progenitor thereof based on the detection or non-detection of the DNA tag in DNA extracted from the organism of interest, or the parasite or the symbiont with the organism of interest.

SEQUENCE LISTING

<110> Japan Software Management Co.,Ltd.

<120> Method for identifying organisms by DNA tag

<130> FP-097PCT

<150> JP 2007/011739
<151> 2007-01-21

<160> 1

<170> PatentIn Ver. 2.1

<210> 1
<211> 88
<212> DNA
<213> Artificial Sequence

<220>
<223> Description of Artificial Sequence:Synthetic DNA

<400> 1
tcgggctata tcctatcggg cgcagcggtc tattgatagg ccgcctccca
gaatgtattg 60
agccggccgc cggctaggta aggcataa 88

## Description

### Field of the Invention

The present invention relates to a method for the identification of an organism by using a DNA tag, more specifically a method for the identification of an individual body, a species, a habitat or the like of an organism by detecting a specific nucleotide sequence(s), still more specifically a method for the identification of an individual body, a species, a habitat or the like of an organism by adding a specific nucleotide sequence(s) in advance to a subj ect of interest artificially and then detecting the nucleotide sequence (s) in the subject. Nucleotide sequences of DNA in organisms vary by individuals, species, habitats or the like of the organisms. Therefore, the identification method of the present invention enables to identify an individual body, a species, a habitat or the like of an organism. In recent years, forged seals attached on foods or the contamination of genetically modified materials into foods become problems of public concern. The identification method of the present invention can be utilized for the detection of the presence or absence of the forged seals or the contamination or the like.

### Background Art

In recent years, from the viewpoint of securing the reliability and safety of foods, it has been increasingly demanded to identify the production areas or cultivars of agricultural products. However, it is impossible to identify the production areas or cultivars of products merely by the observation of their appearance, and it is often the case that the current identification methods can produce no definite result. Under these circumstances, identification methods using DNA have started to be employed recently (see, for example, Non-Patent Reference No. 1).

In the current identification method for the production area or cultivar of an organism using DNA, it is needed to search and determine a DNA region in advance which varies in its nucleotide sequence depending on the production areas or cultivars. For actual identification of the production area or cultivar of an organism of interest, it is needed to analyze the nucleotide sequence of the DNA region in the organism by a method such as nucleotide sequencing and PCR-RFLP and the like, and determine which nucleotide sequence corresponding to which production area or cultivar the obtained nucleotide sequence is identical with. In the conventional identification methods, among various regions of DNA which occur originally in an organism to be examined, a region having a distinct nucleotide sequence from that of another organism is identified as a DNA region which can be used for the identification (see, for example, Patent Reference Nos. 1 and 2). Specifically, the identification of a DNA region which can be used for the identification of strawberry cultivars can be achieved as follows. For example, many fruits of a strawberry cultivar "Toyonoka" are provided, and DNA is extracted from the fruits separately and digested with various restriction enzymes. The digestion products are subjected to electrophoresis separately to obtain their digestion patterns. Next, from many fruits of a strawberry cultivar "Tochiotome", which is examined to be distinguished from "Toyonoka", DNA is extracted separately and digested with various restriction enzymes. The digestion products are subjected to electrophoresis separately to obtain their digestion patterns. Subsequently, the comparison is made on the restriction enzyme digestion pattern between the cultivars "Toyonoka" and "Tochiotome" to find a distinct digestion pattern between these two cultivars. The digestion pattern with a given restriction enzyme is distinct between the cultivars "Toyonoka" and "Tochiotome", because the location of a nucleotide sequence digestable with the restriction enzyme is different between these two cultivars. Then, the DNA region having a distinct digestion pattern is identified as a region which can be employed for the discrimination between these cultivars. In this manner, for determining a DNA region which can be employed for the identification of the production areas or cultivars, enormous time, cost and labor are required. In the actual identification of the production area or cultivar of an organism of interest, DNA extracted from the organism is digested with a restriction enzyme which can provide distinct digestion patterns between the cultivars "Toyonoka" and "Tochiotome", and the digestion product is subjected to electrophoresis to produce a digestion pattern. It is then determined which digestion pattern the organism shows, a digestion pattern for "Toyonoka" or a digestion pattern for "Tochiotome", thereby determining which cultivar the organism belongs, "Toyonoka" or "Tochiotome". As mentioned above, in the first step for the actual identification of the production region or cultivar of the organism, the same procedures are employed as those employed for determining an area which can be used for the discrimination between the cultivars "Toyonoka" and "Tochiotome" as mentioned above.

On the other hand, for the gene therapy or cultivar improvement by genetic recombination which is currently conducted on a trial basis, a variety of techniques have been developed and gone into actual use, which enable to introduce a gene or a gene expression unit having a useful function into an organism of interest, maintain the gene or the gene expression unit in the organism stably, and detect the gene or the gene expression unit maintained in the organism (see, for example, Non-patent Reference Nos. 2 to 4).
[Patent Reference No. 1] Japanese Patent Application Laid-open No. 2002-112774
[Patent Reference No. 2] Japanese Patent Application Laid-open No. 2004-344004
[Non-patent Reference No. 1] Press Release on June 21, 2006, from the Ministry of Agriculture, Forestry and Fisheries, Japan; In "Research Results of the Identification of Cultivars by DNA Analysis in the Test on Rice and Agricultural Products Harvested in 2005." < http://www.maff.go.jp/www/press/2006/20060721press 3.pdf>
[Non-patent Reference No. 2] Sambrook J & Russell DW: Chapter 16 Introducing Cloned Genes into Cultured Mammalian Cells. Molecular Cloning: A laboratory manual. 3rd Ed. 2001; pp16.1-16.62.
[Non-patent Reference No. 3] Sambrook J & Russell DW: Protocol: Introduction to Southern hybridization (Protocols 8-10) In: Chapter 6 Preparation and analysis of eukaryotic genomic DNA. Molecular Cloning : A laboratory manual. 3rd Ed. 2001; pp6. 33-6. 38 . [Non-patent Reference No. 4] Sambrook J & Russell DW: Protocol 28 Screening bacterial colonies by hybridization: Small numbers. In: Chapter 1 Plasmids and their usefulness in Molecular Cloning. Molecular Cloning : A laboratory manual. 3rd Ed. 2001; pp1.1-1.142.

### Disclosure of the Invention

### Problems to be Solved by the Invention

A method currently employed for the identification using DNA comprises the steps of analyzing the nucleotide sequence of DNA of an organism of interest and the nucleotide sequence of DNA of other organism separately, comparing the both nucleotide sequences to each other, identifying a nucleotide sequence specific to the organism of interest, and detecting the presence of the specific DNA sequence. The method is needed to analyze and compare a wide variety of nucleotide sequences, and therefore enormous labor, time and cost are required for the operations for the analysis and the comparison.

The object of the present invention is to provide a novel organism identification method which can be used as an alternative to the above-mentioned identification method which requires enormous labor.

### Means for Solving the Problems

The present inventors have made intensive and extensive studies for the purpose of overcoming the problems as mentioned above. As a result, the inventors have found that the labor, time and cost required can be reduced remarkably compared to the conventional method, by setting a nucleotide sequence of specific DNA as a DNA tag and then detecting the presence of the specific nucleotide sequence to facilitate the determination and identification of a nucleotide sequence specific to the organism to be identified. Based on this finding, the present invention has been accomplished.

Thus, the present invention provides the following items (1) to (7).
(1) An organism identification method for determining whether or not an organism of interest is identical to a specific organism or a progeny thereof, which is characterized by introducing a DNA tag(s) in advance into a cell (s) of the specific organism, or a parasite or a symbiont with the specific organism, and determining whether or not the organism of interest is identical to the specific organism or a progenitor thereof based on the detection or non-detection of the DNA tag in DNA extracted from the organism of interest, or the parasite or the symbiont with the organism of interest.
(2) The organism identification method according to item (1), wherein the introduction of the DNA tag(s) into the cell(s) is the insertion of the DNA tag(s) into the genomic DNA.
(3) The organism identification method according to item (2), wherein the DNA tag(s) is inserted into a site(s) having no function in the genomic DNA.
(4) The organism identification method according to item (2) or (3), wherein one DNA tag is inserted into one or more sites in the genomic DNA.
(5) The organism identification method according to item (2) or (3), wherein two or more DNA tags are inserted into one or more sites in the genomic DNA.
(6) The organism identification method according to any one of items (2) to (5), wherein the DNA tag(s) has a nucleotide sequence(s) which does not occur originally in the genomic DNA of an organism into which the DNA tag(s) is to be introduced.
(7) The organism identification method according to any one of items (2) to (5), wherein the DNA tag(s) has a nucleotide sequence(s) which occurs originally in the genomic DNA of an organism into which the DNA tag(s) is to be introduced and the DNA tag(s) is introduced into a site(s) where the nucleotide sequence(s) does not occur originally in the organism.

### Effect of the Invention

According to the method of the items (1) and (2), a DNA tag having a specific nucleotide sequence is introduced into a specific organism in advance, whereby it becomes possible to identify the nucleotide sequence of DNA to be used for the identification without enormous labor, time and cost. Further, a DNA tag(s) having a specific nucleotide sequence(s) is introduced into a parasite or a symbiont with a specific organism in advance, the parasite or the symbiont is allowed to transfer to the specific organism, and the DNA tag (s) is detected, whereby it becomes possible to introduce the DNA tag(s) indirectly even in the case where it is difficult to directly introduce the DNA tag into the specific organism.

According to the method of the item (3), a DNA tag may be inserted into a site having no function in the genomic DNA of a specific organism, whereby it becomes possible to attach the DNA tag(s) without altering any property of the specific organism.

According to the method of the item (4), one DNA tag may be introduced into two or more sites in the genomic DNA of a specific organism, whereby it becomes possible to develop a variety of patterns of the DNA tag depending on the type of the DNA tag or sites where the DNA tag is to be introduced, and also becomes possible to utilize the patterns of the DNA tag for identifying a variety of target subjects, respectively.

According to the method of the item (5), two or more DNA tags may be inserted into two or more sites in the genomic DNA of a specific organism, whereby it becomes possible to develop a variety of patterns of the DNA tag depending on the number of copies of the DNA tags or sites where the DNA tags are to be introduced, and also becomes possible to utilize the patterns of the DNA tag for identifying a variety of target subjects, respectively. Further, even when some of the DNA tags are detached by accidental recombination or the like, the identification can be achieved by utilizing the remaining DNA tags.

According to the method of the item (6), a nucleotide sequence (s) of DNA which does not occur originally in a specific organism may be used as a DNA tag(s), whereby it becomes possible to identify the presence of the DNA tag in the specific organism clearly.

According to the method of the item (7), a nucleotide sequence(s) of a part(s) of DNA which occurs originally in a specific organism may be used as a DNA tag(s), whereby it becomes possible to define the DNA tag with reduced labor, time and cost, since the need of developing a novel nucleotide sequence is eliminated. Further, the DNA tag(s) may be inserted into a site (s) which does not occur originally in the specific organism, whereby it becomes possible to determine whether or not a sequence(s) located at the site is the DNA tag itself merely by examining the location where the sequence is inserted.

### Brief Description of the Drawing

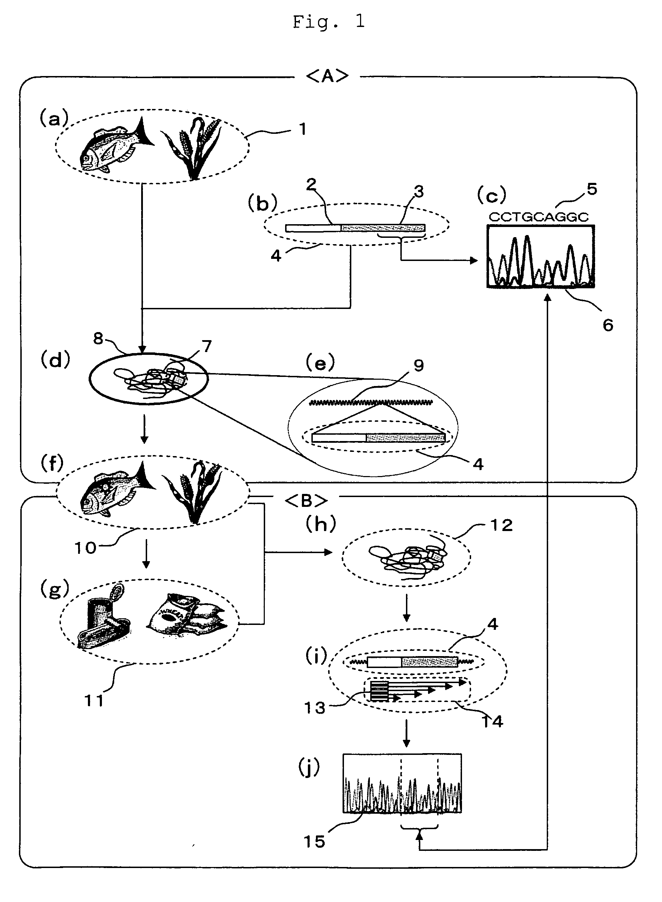

Fig. 1 illustrates the flow of the identification method, in which a DNA tag is inserted into DNA of a specific organism and then the presence of the DNA tag in the specific organism is detected. In Fig. 1, the reference numbers indicate as follows: 1: a specific organism; 2: sequence-primer-binding sequence segment; 3: a tag information sequence segment; 4: a DNA tag; 5: a tag information sequence; 6: a sequence pattern of the tag information sequence; 7: the genomic DNA having the DNA tag inserted therein; 8: a cell carrying the genomic DNA having the DNA tag inserted therein; 9: a part of the genomic DNA of the specific organism; 10: an organism to be identified; 11: a processed product containing the organism to be identified as a raw material; 12: the genomic DNA extracted from the organism to be identified; 13: the sequence primer; 14: a sequence fragment; and 15: a sequence pattern of the organism to be identified.

### Best Mode for Carrying Out the Invention

Hereinbelow, the present invention will be described in detail.

The organism identification method according to the present invention is a method for determining whether or not an organism of interest is identical to a specific organism or a progeny thereof, which is characterized by introducing a DNA tag(s) in advance into a cell of the specific organism, or a parasite or a symbiont with the specific organism, and determining whether or not the organism of interest is the specific organism or a progenitor thereof based on the detection or non-detection of the DNA tag in DNA extracted from the organism of interest, or the parasite or the symbiont with the specific organism of interest.

The specific organism can be selected arbitrarily by a practitioner of the identification method of the present invention, and may be a specific individual body of an organism, an organism belonging to a specific taxonomic group (e.g. , a cultivar, a breed, a species, a genus), an organism inhabiting a specific habitat, or the like. The specific organism may be any one of an animal, a plant, a microorganism or the like, or may be any one of a multicellular organism or a unicellular organism.

The cell into which the DNA tag is to be introduced is not particularly limited, and is preferably such a cell that the DNA tag introduced into the cell can be transmitted to a progeny of an organism carrying the cell. For an animal, examples of the cell include a fertilized egg, a sperm cell and an egg. For a plant, the cell may be one capable of being differentiated into an individual organism, such as a cell capable of forming a callus.

In general, the DNA tag is introduced into the specific organism. However, it is also possible to introduce the DNA tag into an organism which is parasitic in or can live symbiotically with the specific animal. Examples of the combination of the specific organism and the organism parasitic therein or the like include a combination of a *Porphyra* and an alphaproteobacterium which can live symbiotically with the *Porphyra,* a combination of an aphid and a buchnera which can live symbiotically with the aphid, a combination of *Mesodinium,* which can generate red tides and a Cryptophyte alga which can live symbiotically with the *Mesodinium,* and a combination of any virus and a host parasitized with the virus.

The technique for introducing the DNA tag into the cell is not particularly limited, and is preferably a method for inserting the DNA tag into the genomic DNA. Examples of the method include a method utilizing homologous recombination and a method utilizing a vector enabling the integration of DNA into a genome. The method utilizing homologous recombination (for an animal genome: Paul D. Richardson et al., Gene Repair and Transposon-Mediated Gene Therapy. Stem Cells : 2002;20:105-118; for a plant cell: Endo M et al., Increased frequency of homologous recombination and T-DNA integration in Arabidopsis CAF-1 mutants. EMBO J. 2006 Nov 29;25(23):5579-90. Epub 2006 Nov 16.) and the method utilizing a vector enabling the integration of DNA into a genome (for a lentivirus: Bryda EC et al., Method for detection and identification of multiple chromosomal integration sites in transgenic animals created with lentivirus. Biotechniques : 2006, Dec 41 (6) :715-9; and for a retrovirus: Koo, B.C. et al., Production of germline transgenic chickens expressing enhanced green fluorescent protein using a MoMLV-based retrovirus vector. FASEB J. : 2006, Nov; 20(13):2251-60) are well-known. Therefore, a person skilled in the art can insert the DNA tag into the genomic DNA by employing any one of these methods. The technique for introducing the DNA tag into the cell is not limited to a method for inserting the DNA tag into genomic DNA, and the DNA tag may be introduced into the cell in such a manner that the DNA tag is present in the cell in an episomal state. Such introduction method is also well-known (for an Epstein-Barr virus: Ren, P.et al., Establishment and Applications of Epstein-Barr Virus-Based Episomal Vectors in Human Embryonic Stem Cells. Stem Cells : 2006, 24 (5) 1338-1347), and a person skilled in the art can introduce the DNA tag into the cell by employing this method.

For the insertion of the DNA tag into the genomic DNA, the nucleotide sequence, the number and the length of the DNA tag(s), the nature and the number of the insertion site(s), the mode of insertion, and the detection method are critical seven matters theoretically.

With regard to the nucleotide sequence of the DNA tag, the choice between the following two alternatives is theoretically possible: a nucleotide sequence (s) which occurs originally in the specific organism, a parasite or a symbiont with the specific organism (also referred to as a "specific organism or the like", hereinafter) is used; or a nucleotide sequence(s) which does not occur originally in the specific organism or the like is used. In the case where the whole nucleotide sequence of DNA of the specific organism or the like is already analyzed, a person skilled in the art can confirm readily as to whether or not the nucleotide sequence of specific DNA occurs in the specific organism or the like by searching on the homology between the nucleotide sequence date for the DNA and the nucleotide sequence of DNA which is intended to be used as the DNA tag using a homology search software known to a person skilled in the art (see, for example, DNA Data Bank of Japan, "Explanation of a software FASTA for homology search" http://www.ddbj.nig.ac.jp/search/explain/fasta txt-j.html). In the case where the whole nucleotide sequence of DNA of the specific organismor the like is not analyzed yet, the confirmation as to whether or not the nucleotide sequence of a specific DNA occurs in the specific organism or the like can be made by a molecular biological basic experimental procedure such as Southern blot hybridization or dot blot hybridization, without the need of detail analysis on the nucleotide sequence of DNA of the specific organism of the like (see, for example, Sambrook J & Russell DW Protocol: Introduction to Southern hybridization (Protocols 8-10): Chapter 6 Preparation and analysis of eukaryotic genomic DNA. Molecular Cloning: A laboratory manual. 3rd Ed. 2001; pp6.33-6.38.; and the Documentation Division of the Japanese Patent Office, other information on references, Standard techniques (Hyojun Gijutsu-shu), Amplification and Detection of Nucleic acid, 2-2-5: Southern hybridization https://www.jpo.go.jp/shiryou/s sonota/hyoujun gijutsu/kakusa n/0018.html). With regard to the nucleotide sequence of the DNA tag, there is a choice whether or not to have any function (e.g., a structural gene, a promoter, an enhancer). It is possible to choose between a sequence having any function and a sequence having no function. When the nucleotide sequence having any function is used, the sequence may cause any change in a phenotype(s) of the specific organism or the like. Therefore, it is preferred to choose a sequence having no function.

The number of the DNA tags can be select arbitrarily. Only one DNA tag may be inserted, or two or more DNA tags may be inserted. The length of the DNA tag can also be selected arbitrarily depending on the DNA detection method employed as mentioned below, and is preferably several to several thousands nucleotides, more preferably 30 to 1,000 nucleotides.

With regard to the site into which the DNA tag is to be inserted, the choice between the following two alternatives is theoretically possible: the DNA tag is inserted into a site having any function in the specific organism or the like; and the DNA tag is inserted into a site having no function in the specific organism or the like. However, when the DNA tag is inserted into a site having any function in the specific organism or the like, there is a high risk that a function inherent in the site may be eliminated and any change in properties may occur in the organism. Therefore, such a site is not proper as a site to be inserted with a DNA tag which is used for identification. Practically, it is believed to be desirable to insert the DNA tag into a site having no function in the specific organism or the like. In general, it is difficult to demonstrate that a given site has no function in the specific organism or the like. However, for example, there are some pseudogenes in an organism which are known to completely lose their functions (see, for example, Julie R. Wafaei and Francis Y.M. Choy, Glucocerebrosidase recombinant allele: molecular evolution of the glucocerebrosidase gene and pseudogene in primates. Blood Cells Mol Dis. : 2005 Sep-Oct;35(2):277-85). When the DNA tag is inserted into the site, the DNA tag can be added without deteriorating any original function of the specific organism or the like. Besides a site corresponding to a pseudogene, any other site having no function can be considered as a candidate site for insertion. One or more of such sites can be selected and employed as the insertion sites. Even when the site to be inserted has any function, the DNA tag can be inserted into a position in the site which does not affect its function. An example of the position is a position to which a His-tag can be added in various His-tag fusion proteins used for the purification of a protein synthesized *in vitro* (see, for example, Mathur D & Garg LC. Functional phosphoglucose isomerase from Mycobacterium tuberculosis H37Rv: Rapid purification with high yield and purity. Protein Expr Purif. 2006 Oct 25 [Epub ahead of print]).

Predicated on the requirements mentioned above, the number of insertion sites can be selected arbitrarily. With regard to the mode of insertion, the following modes can be employed in any combination theoretically: a mode in which only one DNA tag is inserted into a specific one site or specific two or more sites; a mode in which two or more of the same or different DNA tags are inserted into sites by one tag per one site; and a mode in which a single sequence produced by linking two or more of the same or different DNA tags together with varying directions or numbers of the DNA tags is inserted into one or more sites.

As for the method for detecting the DNA tag, when a nucleotide sequence which does not occur originally in the specific organism or the like is used as the DNA tag, DNA is extracted from the specific organism or the like having the DNA tag inserted therein and it is then determined whether or not a nucleotide sequence specific to the DNA tag is contained in the extracted DNA. The extraction of DNA from the specific organism or the like can be achieved by a person skilled in the art readily by employing any one of various known procedures (see, for example, Sambrook J & Russell DW Protocol: Introduction to Southern hybridization (Protocols 8-10): Chapter 6 Preparation and analysis of eukaryotic genomic DNA. Molecular Cloning: A laboratory manual. 3rd Ed. 2001; pp6. 33-6. 38) . The determination whether or not a nucleotide sequence specific to the DNA tag is contained in the extracted DNA can be achieved by a person skilled in the art readily by employing any one of known basic molecular biological experimental procedures, such as Southern blot hybridization or dot blot hybridization using a nucleotide sequence of the DNA tag(s) as a probe(s) (see, for example, Sambrook J & Russell DW Protocol: Introduction to Southern hybridization (Protocols 8-10): Chapter 6 Preparation and analysis of eukaryotic genomic DNA. Molecular Cloning: A laboratory manual. 3rd Ed. 2001; pp6.33-6.38.), or nucleotide sequencing method (see, for example, Sambrook J & Russell DW: Chapter 12 DNA sequencing. Molecular Cloning: A laboratory manual. 3rd Ed. 2001; pp12.1-12.120).

When a nucleotide sequence which originally occurs in the specific organism or the like is used as the DNA tag, examples of the method for detecting the DNA tag include the following methods: a method in which Southern blot hybridization of DNA extracted from the specific organism or the like is carried out by using a nucleotide sequence of the DNA tag as a probe, and the detection is then made on whether or not the probe can hybridize with multiple electrophoresis bands (see, for example, M Yoshida, M Seiki, K Yamaguchi, and K Takatsuki: Monoclonal integration of human T-cell leukemia provirus in all primary tumors of adult T-cell leukemia suggests causative role of human T-cell leukemia virus in the disease. Proc Natl Acad Sci U S A. 1984 April; 81 (8) : 2534-2537): a method in which PCR is carried out by using, as a primer, a nucleotide sequence located adjacent to the insertion site and outside of the DNA tag inserted, and electrophoresis of an amplification product of the PCR is carried out to detect the presence of a fragment having such a length that can contain the DNA tag (see, for example, S Murata, N Takasaki, M Saitoh, and N Okada : Determination of the phylogenetic relationships among Pacific salmonids by using short interspersed elements (SINEs) as temporal landmarks of evolution. Proc Natl Acad Sci USA. 1993 August 1; 90(15): 6995-6999); a method in which PCR is carried out in the same manner, a double-strand-specific fluorescent intercalator is reacted with products of the PCR, and it is confirmed that the product emits more intense fluorescence because a fragment having a larger length than a PCR product which does not contain the DNA tag is amplified; a method in which real time PCR is carried out using, as a primer, a nucleotide sequence adjacent to the insertion site and outside of the DNA tag inserted, and it is confirmed that a product of the real time PCR emits more intense fluorescence because a fragment having a larger length than a PCR product which does not contain the DNA tag is amplified; and the like. A person skilled in the art can detect the DNA tag readily by using any one of these known molecular biological procedures.

The basic concept of one embodiment of the identification method of the present invention will be explained with reference to Fig. 1, in which a nucleotide sequence which does not occur originally in a specific organism is inserted in advance into the genomic DNA of the specific organism as the DNA tag, and it is determined whether or not the specific organism carries the nucleotide sequence. The identification method comprises the following two steps: a first step for inserting the DNA tag in advance into the specific organism; and a second step for determining whether or not an organism of interest carries the DNA tag. In Fig. 1, the first step and the second step are illustrated in the part <A> and the part <B>, respectively.

The first step for inserting the DNA tag into the specific organism will be described. As an example, the explanation will be made on a case where the tag is inserted into genomic DNA of a specific organism (1) as illustrated in Fig. 1 (a). First, a fertilized egg (for an animal), a cell of a calluse (for a plant) or the like of a specific organism (1) is provided. The cell or the like can be prepared by a person skilled in the art readily. A nucleotide sequence of a DNA tag (4) is selected from nucleotide sequences which do not originally occur in the genomic DNA of the specific organism (1) as illustrated in (b), and DNA having the nucleotide sequence is produced. The production of the DNA can be achieved by any one of molecular biological procedures known to a person skilled in the art. For example, in the case where the DNA tag is short, a chemical synthesis method can be employed, in which single strands of the DNA tag are separately synthesized chemically and the synthesized single strands are annealed together to form a double-stranded molecule. In the case where a suitable template DNA molecule can be provided to produce a relatively long DNA tag, the DNA tag can be produced by polymerase chain reaction (PCR: see, for example, Mullis K and other five persons, Specific enzymatic amplification of DNA in vitro: The polymerase chain reaction. Cold Spring Harb Symp Quant Biol. 1986; 51 Pt 1:263-73) . The DNA tag (4) has such a structure that a sequence segment having a sequence primer bound thereto (hereinafter, also referred to as a "sequence-primer-binding sequence segment") (2) is linked to the 3'-end of a tag information sequence segment (3). The sequence-primer-binding sequence segment (2) has a nucleotide sequence complementary to a sequence primer (13), and can serve as a segment to which the sequence primer is bound when the DNA tag is detected by nucleotide sequencing. The tag information sequence segment (3) is a relatively short nucleotide sequence which does not occur originally in the genomic DNA extracted from the specific organism. The tag information sequence segment (3) has a nucleotide sequence such as a tag information sequence shown in (c), and shows a pattern like a sequence pattern (6) of the tag information sequence as determined by the analysis of the nucleotide sequence thereof by capillary electrophoresis. The DNA tag (4) having the structure mentioned above is inserted into the genomic DNA in a cell (e.g., a fertilized egg (for an animal), a cell of a calluse (for a plant)) of the specific organism (1) or the like by *in vitro* homologous recombination or the like (see, for example, Paul D. Richardson and other three persons, Gene Repair and Transposon-Mediated Gene Therapy. Stem Cells : 2002;20:105-118), thereby producing a cell (8) carrying the genomic DNA (7) having the DNA tag inserted therein (d). The enlarged view of the site at which the DNA tag is inserted is as illustrated in (e), in which the DNA tag (4) is inserted into a part of the genomic DNA (9) of the specific organism. An individual body carrying the genomic DNA (7) having the DNA tag (4) inserted therein is produced from the cell (8) carrying the genomic DNA (7) having the DNA tag inserted therein. This is the first step for inserting a DNA tag into a specific organism.

Next, the second step for determining whether or not an organism of interest has the DNA tag, as illustrated in the part <B> in Fig. 1, will be described. The genomic DNA (12) is extracted (h) from an organism to be identified (10) such as an organism illustrated in (f) or a processed product (11) containing the organism to be identified as a raw material such as a product illustrated in (g). Any molecular biological procedure known to a person skilled in the art may be employed for the extraction, as long as it is suitably applicable to the material to be used for the extraction (see, for example, Sambrook J & Russell DW; Chapter 6 Preparation and analysis of eukaryotic genomic DNA. Molecular Cloning: A laboratory manual. 3rd Ed. 2001; pp6.1-6.64; and Sambrook J & Russell DW: Chapter 7 Extraction, purification, and analysis of mRNA from eukaryotic cells. Molecular Cloning: A laboratory manual. 3rd Ed. 2001; pp7.1-7.94). If the organism (10) to be identified has the DNA tag (4) therein, then the sequence-primer-binding sequence segment (2) is contained in the DNA tag (4). Then, a sequence reaction is carried out by using a sequence primer (13) carrying a nucleotide sequence complementary to a part of the sequence-primer-binding sequence segment (2). A sequence fragment (14) which is useful for the analysis of the nucleotide sequence of the tag information sequence segment (3) is produced to carry out the nucleotide sequencing. A sequence pattern (15) for the organism (10) to be identified obtained by the sequencing is compared to the sequence pattern (6) for the tag information sequence to determine whether or not the sequence pattern (6) for the tag information sequence is contained in the sequence pattern (15) for the organism (10) to be identified. It is determined as that the organism (10) to be identified or the processed product (11) containing the organism (10) as a raw material contains the DNA tag (4) when the sequence pattern (6) for the tag information sequence is contained in the sequence pattern (15) for the organism (10).

### Example

Hereinafter, the present invention will be described great in detail with reference to the following example. However, the present invention is not limited by the following example.

For finding a candidate nucleotide sequence for a useful DNA tag, search was made for a sequence which does not occur in the genome of a plant *Arabidopsis thaliana* whose whole genomic nucleotide sequence is already open to the public. As for the database for *Arabidopsis thaliana*, the whole genome information provided by the Arabidopsis Information Resource (TAIR) (http://www.arabidopsis.org/) is used. As a result of the search, a nucleotide sequence depicted in SEQ ID NO:1 is found as a sequence which does not occur in the genome of *Arabidopsis thaliana*. It is found that a nucleotide sequence useful as the DNA tag can be found in this manner.

The specification includes the contents as described in the specification and/or drawings of Japanese Patent Application No. 2007-011739, which is a priority document of the present application. All publications, patents and patent applications cited herein are incorporated herein by reference in their entirety.

## Claims

1. An organism identification method for determining whether or not an organism of interest is identical to a specific organism or a progeny thereof, which is **characterized by** introducing a DNA tag(s) in advance into a cell(s) of the specific organism, or a parasite or a symbiont with the specific organism, and determining whether or not the organism of interest is identical to the specific organism or a progenitor thereof based on the detection or non-detection of the DNA tag in DNA extracted from the organism of interest, or the parasite or the symbiont with the organism of interest.

2. The organism identification method according to claim 1, wherein the introduction of the DNA tag(s) into the cell(s) is the insertion of the DNA tag(s) into the genomic DNA.

3. The organism identification method according to claim 2, wherein the DNA tag(s) is inserted into a site(s) having no function in the genomic DNA.

4. The organism identification method according to claim 2 or 3, wherein one DNA tag is inserted into one or more sites in the genomic DNA.

5. The organism identification method according to claim 2 or 3, wherein two or more DNA tags are inserted into one or more sites in the genomic DNA.

6. The organism identification method according to any one of claims 2 to 5, wherein the DNA tag(s) has a nucleotide sequence (s) which does not occur originally in the genomic DNA of an organism into which the DNA tag(s) is to be introduced.

7. The organism identification method according to any one of claims 2 to 5, wherein the DNA tag (s) has a nucleotide sequence (s) which occurs originally in the genomic DNA of an organism into which the DNA tag(s) is to be introduced and the DNA tag(s) is introduced into a site(s) where the nucleotide sequence(s) does not occur originally in the organism.
